(19) Europäisches Patentamt
European Patent Office
Office européen des brevets

(11) Numéro de publication: **0 518 753 B1**

# (12) FASCICULE DE BREVET EUROPEEN

(45) Date de publication de fascicule du brevet: **14.12.94**

(51) Int. Cl.$^5$: **C07C 69/013**, C07C 35/205

(21) Numéro de dépôt: **92401591.0**

(22) Date de dépôt: **10.06.92**

## (54) Nouveaux dérivés de 1,5-diyne-3-cycloalcène, leur procédé de préparation et les compositions pharmaceutiques qui les contiennent.

(30) Priorité: **11.06.91 FR 9107045**

(43) Date de publication de la demande:
**16.12.92 Bulletin 92/51**

(45) Mention de la délivrance du brevet:
**14.12.94 Bulletin 94/50**

(84) Etats contractants désignés:
**AT BE CH DE DK ES FR GB GR IT LI LU NL PT SE**

(56) Documents cités:

**Aucun document pertinent revelé**

(73) Titulaire: **ADIR ET COMPAGNIE**
**1 rue Carle Hébert**
**F-92415 Courbevoie Cédex (FR)**

(72) Inventeur: **Beau, Jean-Marie**
**Les Foucault**
**F-45240 Menestreau en Villette (FR)**
Inventeur: **Crevisy, Christophe, La Renaudine**
**Rue des Ecoles,**
**PERRIGNY**
**F-89000 Auxerre (FR)**
Inventeur: **Atassi, Ghanem**
**4 rue Joséphine**
**F-92400 Saint Cloud (FR)**
Inventeur: **Pierre, Alain**
**52 rue de Montval**
**F-78160 Marly le Roi (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux dérivés de 1,5-diyne-3-cycloalcène, leur procédé de préparation et les compositions pharmaceutiques qui les contiennent.

Les esperamicines et les calicheamicines appartiennent à deux familles d'antibiotiques naturels possédant des propriétés antitumorales nettement plus puissantes que celle des composés anticancéreux connus comme décrit par B. LONG et coll. (Proc. Natl. Acad. Sci., 86, 2-6 1989).

Les propriétés antitumorales de ces deux séries de composés sont dues à la présence d'un noyau 1,5-diyne-3-cycloalcène commun à ces deux familles (Nature, 389, 566-567, 1991).

La présente invention a pour objet des dérivés de 1,5-diyne-3-cycloalcène qui outre le fait qu'ils soient nouveaux présentent des propriétés antitumorales particulièrement intenses.

L'invention concerne plus particulièrement de nouveaux dérivés de 1,5-diyne-3-cycloalcène répondant à la formule générale (I) :

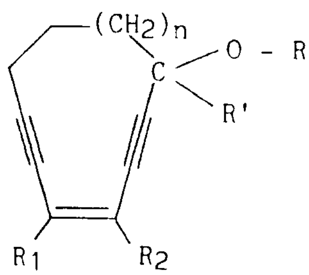

(I)

dans laquelle :

R représente un atome d'hydrogène, un radical acyl ($C_1$-$C_6$) linéaire ou ramifié ou un radical glycoside,

R' représente un atome d'hydrogène ou

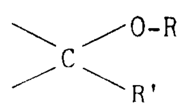

représente

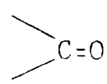

n est égal à 1 ou 2,

$R_1$ et $R_2$ représentent simultanément deux atomes d'hydrogène ou forment avec la double liaison à laquelle ils sont attachés un noyau phényle,

leurs isomères et énantiomères.

L'invention s'étend aussi au procédé de préparation des composés de formule (I) caractérisé en ce que l'on fait réagir, en milieu anhydre, sous atmosphère inerte, le (Z)-1,2-dichloroéthylène ou le 1,2-dibromobenzène avec un alcool de formule (II) :

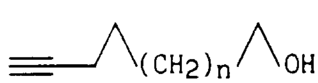

(II)

dans laquelle n a la même signification que dans la formule (I), en présence d'un catalyseur palladié préparé à partir de tétrakis (triphénylphosphine) palladium, de n-propylamine et d'iodure de cuivre, selon les techniques décrites par K. SONOGASHIRA et coll. (Tetrahedron lett. 4467-4470, 1975) et D. GUILLERM (Tetrahedron lett., 26, 3811-3812, 1985),

pour conduire au composé de formule (III) :

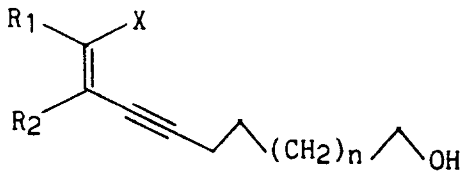

(III)

dans laquelle n, $R_1$ et $R_2$ ont la même signification que dans la formule (I) et X représente un atome de chlore ou de brome selon le produit de départ utilisé,
qui est couplé, comme précédemment, en présence de tétrakis (triphénylphosphine) palladium, de n-propylamine et d'iodure de cuivre, avec du triméthylsilylacétylène, en milieu anhydre, sous atmosphère inerte,
pour conduire au composé de formule (IV) :

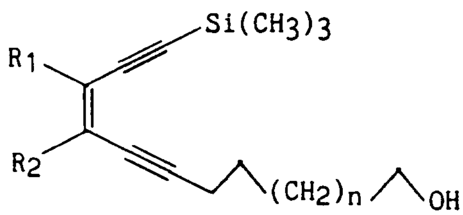

(IV)

dans laquelle n, $R_1$ et $R_2$ ont la même signification que dans la formule (I),
que l'on soumet à une désilylation en milieu basique anhydre, sous atmosphère inerte,
pour conduire au composé de formule (V) :

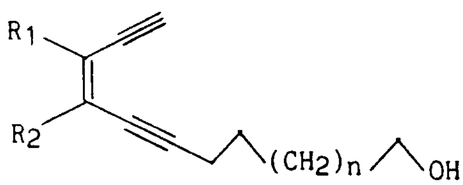

(V)

dans laquelle n, $R_1$ et $R_2$ ont la même signification que dans la formule (I),
sur lequel on fait réagir de l'iode en milieu organique anhydre en présence de morpholine, sous atmosphère inerte,
pour conduire au composé de formule (VI) :

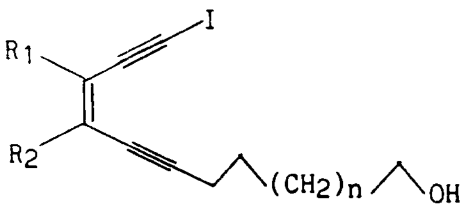

(VI)

dans laquelle n, $R_1$ et $R_2$ ont la même signification que dans la formule (I),
que l'on soumet à une oxydation en présence de chlorochromate de pyridinium en milieu organique anhydre, sous atmosphère inerte,
pour conduire au composé de formule (VII) :

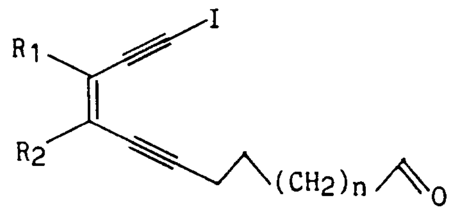

(VII)

dans laquelle n, $R_1$ et $R_2$ ont la même signification que dans la formule (I), que l'on cyclise à l'aide d'un mélange de chlorure de chrome contenant 1,3 % de chlorure de nickel en suspension dans du tétrahydrofurane anhydre, à température ambiante, sous atmosphère inerte, selon les techniques décrites par K. TAKAI et coll. (Tetrahedron lett., 26, 5585-5588, 1985) et T.D. AICHER (Tetrahedron lett., 28, 3463-3466, 1987),
pour conduire au composé de formule (I/a), cas particulier des composés de formule (I) :

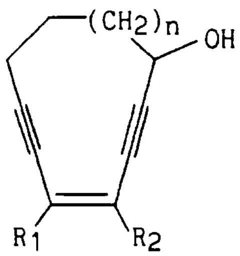

(I/a)

dans laquelle n a la même signification que dans la formule (I), qui est :
- soit transformé en ester correspondant, sous atmosphère inerte,

pour conduire au composé de formule (I/b), cas particulier des composés de formule (I),

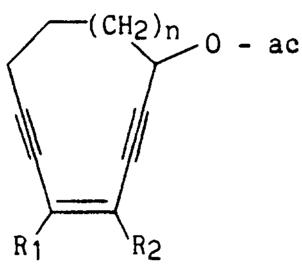

(I/b)

dans laquelle n, $R_1$ et $R_2$ ont la même signification que dans la formule (I) et ac représente un groupement acyl ($C_1$-$C_6$) linéaire ou ramifié,
- soit oxydé en présence de chlorochromate de pyridinium,

pour conduire au composé de formule (I/c), cas particulier des composés de formule (I),

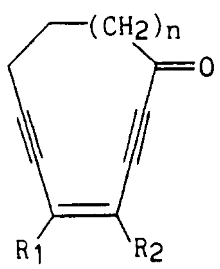

(I/c)

dans laquelle n, $R_1$ et $R_2$ ont la même signification que dans la formule (I),
- soit glycosylé en présence de trichloroacétamidate d'acétylglycoside puis désacétylé après séparation éventuelle des isomères,

pour conduire au composé de formule (I/d), cas particulier des composés de formule (I),

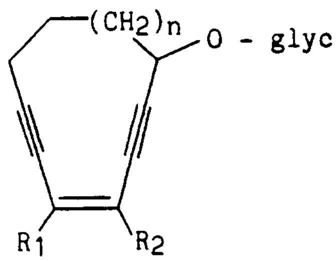

(I/d)

dans laquelle n, $R_1$ et $R_2$ ont la même signification que dans la formule (I) et glyc représente un groupement glycoside,
composés de formule (I/a), (I/b), (I/c) et (I/d), que l'on purifie, le cas échéant, selon une technique classique de préparation et dont on sépare les isomères, si on le souhaite, selon une technique classique de séparation.

Les composés de l'invention possèdent des propriétés pharmacologiques très intéressantes. Ils inhibent la prolifération des cellules L 1210 (leucémie murine) en culture, ce qui est prédictif d'une bonne activité antitumorale chez l'animal et aussi chez l'homme.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les suppositoires, crèmes, pommades, gels dermiques, les aérosols.

La posologie varie selon l'âge et le poids du patient, la nature et la sévérité de l'affection ainsi que la voie d'administration. Celle-ci peut être orale, nasale, rectale ou parentérale. D'une manière générale, elle s'échelonne entre 0,2 et 200 mg pour un traitement en une ou plusieurs prises par 24 heures.

Les exemples suivants illustrent l'invention et ne la limitent en aucune façon.

**EXEMPLE 1 : 1,5-Diyne-(3Z)-cyclodécène-7-ol**

Stade A : 8-Chloro-5-yne-7-octène-1-ol

A une solution de 85 mg (0,074 mmole) de tétrakis (triphénylphosphine) palladium dans 8 ml de benzène anhydre sont successivement ajoutés 650 µl (7,01 mmoles) de n-propylamine, 480 µl (4,26 mmoles) de 5-hexyne-1-ol, 420 µl (5,40 mmoles) de cis-dichloroéthylène et 35 mg (0,184 mmole) d'iodure de cuivre.

Le mélange réactionnel est chauffé pendant 100 minutes à 40°C sous atmosphère inerte puis concentré et repris par de l'éther diéthylique. La phase organique est lavée avec une solution aqueuse saturée de chlorure de sodium, séchée puis évaporée. Le produit attendu est obtenu sous forme d'un liquide incolore après purification par chromatographie sur silice en utilisant comme solvant d'élution un mélange dichlorométhane/acétone (40/1).

Rendement : 78 %

Spectre de masse : ionisation chimique ($NH_3$)

$M + NH_4^+ : m/_z = 176$

Stade B : 10-Triméthylsilyl-5,9-diyne-7-décène-1-ol

516 mg (3,25 mmoles) du composé obtenu au stade A dans 7 ml de benzène anhydre sont traités par 500 µl (6,08 mmoles) de n-propylamine, 70 mg (0,061 mmole) de tétrakis (triphénylphosphine) palladium, 32 mg (0,168 mmole) d'iodure de cuivre et 650 µl (4,51 mmoles) de triméthylsilylacétylène. Le milieu réactionnel est agité 100 minutes, à température ambiante, sous atmosphère inerte, puis concentré et repris par de l'éther diéthylique. La phase organique est lavée par une solution aqueuse saturée de chlorure de sodium puis par de l'eau, séchée et évaporée. Le produit attendu est obtenu, sous forme d'huile, après purification par chromatographie liquide sur silice en utilisant comme solvant d'élution un mélange dichlorométhane/acétone/triéthylamine (40/1/0,04).

Rendement : 82 %

Stade C : 5,9-Diyne-7-décène-1-ol

1,12 g (5,08 mmoles) du composé obtenu au stade B en solution dans 10 ml de méthanol anhydre sont traités par 770 mg (5,57 mmoles) de carbonate de potassium à température ambiante, sous atmosphère inerte, pendant 15 minutes. Le milieu réactionnel est alors concentré, repris par du dichlorométhane et lavé à l'eau. La phase organique est alors séchée et évaporée. Le produit attendu est obtenu, sous forme d'huile incolore, après purification par chromatographie liquide sur silice en utilisant comme solvant d'élution un mélange dichlorométhane/acétone (20/1).
Rendement : 90 %
Spectre de masse : ionisation chimique ($NH_3$)
$M + NH_4^+ : m/_z = 166$

Stade D : 10-Iodo-5,9-diyne-7-décène-1-ol

A une solution contenant 2,42 g (9,53 mmoles) d'iode dans 20 ml de benzène anhydre chauffée à 45°C sont ajoutés 2,49 ml (28,55 mmoles) de morpholine. Après 20 minutes d'agitation à 45°C, sous atmosphère inerte, 675 mg (4,55 mmoles) du composé obtenu au stade C, en solution dans 5 ml de benzène anhydre, sont ajoutés. Le mélange réactionnel est maintenu sous agitation 3 heures à 45°C, concentré, repris par de l'éther diéthylique, et lavé successivement avec une solution aqueuse saturée de chlorure de sodium, une solution aqueuse à 20 % de dihydrogénophosphate de sodium, une solution aqueuse à 20 % de thiosulfate de sodium, une solution aqueuse à 10 % de bicarbonate de sodium puis à l'eau. La phase organique est alors lavée et évaporée. Le produit attendu est alors obtenu, sous forme d'huile, après purification par chromatographie sur silice en utilisant comme solvant d'élution un mélange hexane/éther diéthylique (1/1).
Rendement : 80 %
Spectre de masse : Ionisation chimique ($NH_3$)
$M + NH_4^+ : m/_z = 292$

Stade E : 10-Iodo-5,9-diyne-7-décène-1-al

250 mg (1,16 mmole) de chlorochromate de pyridinium, 2 ml de dichlorométhane sont agités dans du tamis moléculaire (4Å) activé en poudre pendant 20 minutes, à température ambiante, sous atmosphère inerte. 94 mg (0,343 mmole) du composé obtenu au stade D en solution dans 3 ml de dichlorométhane anhydre sont ajoutés au mélange précédent. L'ensemble est maintenu sous agitation 30 minutes, à température ambiante puis 20 ml d'éther diéthylique sont ajoutés. Le milieu réactionnel est alors filtré puis concentré. Le produit attendu est obtenu après purification par chromatographie sur silice en utilisant comme solvant d'élution un mélange hexane/éther diéthylique (1/1).
Rendement : 83 %
Spectre de masse : Ionisation chimique ($NH_3$)
$M + NH_4^+ : m/_z = 290$

Stade F : 1,5-Diyne-3-cyclodécène-7-ol

181 mg (1,47 mmole) de chlorure de chrome contenant 1,3 % de chlorure de nickel en suspension dans 20 ml de tétrahydrofurane sont agités 20 minutes, à température ambiante, sous atmosphère inerte. 78 mg (0,29 mmole) du composé obtenu au stade E en solution dans 9 ml de tétrahydrofurane sont ajoutés très lentement au milieu précédent. Après l'addition qui dure environ 2 heures 40 minutes, le milieu réactionnel est concentré et repris par de l'acétate d'éthyle. La phase organique est lavée par une solution aqueuse saturée de chlorure de sodium, séchée puis évaporée. Le produit attendu est obtenu sous forme d'huile après purification par chromatographie liquide sur silice en utilisant comme solvant d'élution un mélange hexane/acétate d'éthyle/triéthylamine (75/25/0,1).
Rendement : 34 %
Spectre de masse : Ionisation chimique ($NH_3$)
$M + NH_4^+ : m/_z = 164$

**EXEMPLE 2 : 7-Acétoxy-1,5-diyne-3-cyclodécène**

14,6 mg (0,1 mmole) du composé obtenu dans l'exemple 1 sont traités par 1 ml de pyridine et 0,5 ml d'anhydride acétique pendant 45 minutes, à température ambiante, sous atmosphère inerte. Le milieu réactionnel est dilué à l'éther diéthylique, lavé successivement avec de l'eau, une solution aqueuse à 50 % d'hydrogénosulfate de potassium, une solution aqueuse à 50 96 de bicarbonate de sodium puis avec de l'eau. La phase organique est séchée puis évaporée. Le produit attendu est obtenu sous forme d'huile après purification par chromatographie liquide sur silice en utilisant comme solvant d'élution un mélange hexane/acétate d'éthyle/triéthylamine (83/17/0,1).
Rendement : 59 %
Spectre de masse : impact électronique
M : $m/_z$ = 188

**EXEMPLE 3 : 1,5-Diyne-3-cycloundécène-7-ol**

Le produit attendu est obtenu en procédant comme dans l'exemple 1 mais en utilisant au stade A le 6-heptyne-1-ol à la place du 5-hexyne-1-ol.
Rendement (stade F) : 76 %
Spectre de masse : Ionisation chimique ($NH_3$)
M + $NH_4^+$ : $m/_z$ = 178

**EXEMPLE 4 : 7-Acétoxy-1,5-diyne-3-cycloundécène**

Le produit attendu est obtenu en procédant comme dans l'exemple 2 mais en remplaçant le composé de l'exemple 1 par le composé de l'exemple 3.
Rendement : 90 %
Spectre de masse : impact électronique
M : $m/_z$ = 202

**EXEMPLE 5 : 7-Hydroxy-benzo[c]cyclodéca-1,5-diyne**

Le produit attendu est obtenu en procédant comme dans l'exemple 1 mais en remplaçant au stade A le cis-dichloroèthylène par le 1,2-dibromobenzène.
Spectre de masse : impact électronique
M : $m/_z$ = 196

**EXEMPLE 6 : 7-Acetoxy-benzo[c]cyclodéca-1,5-diyne**

Le produit attendu est obtenu en procédant comme dans l'exemple 2 mais en remplaçant le composé de l'exemple 1 par le composé de l'exemple 5.
Rendement : 70 %
Spectre de masse : impact électronique
M : $m/_z$ = 238

**EXEMPLE 7 : 7-Oxo-benzo[c]cyclodéca-1,5-diyne**

Le produit attendu est obtenu par oxydation du composé de l'exemple 5 en présence de chlorochromate de pyridinium.
Rendement : 70 %
Spectre de masse : impact électronique
M : $m/_z$ = 194

**EXEMPLE 8 : 7-(β-D-Glucopyranosyl)benzo[c]cyclodéoa-1,5-diyne, isomère 1**
et
**EXEMPLE 9 : 7-(β-D-Glucopyranosyl)benzo[c]cyclodéca-1,5-diyne, isomére 2**

**EXEMPLE 8 : 7-(β-D-Glucopyranosyl)benzo[c]cyclodéca-1,5-diyne, isomére 1**

Stade A : 7-(β-D-2,3,4,6-tétra-0-acétylglucopyranosyl)benzo[c] cyclodéca-1,5-diyne, isomère 1

413 mmoles du composé obtenu dans l'exemple 5 et de trichloroacétamidate de 2,3,4,6-tétra-O-acétyl-α-D-glucopyranosyl préparé selon le procédé décrit par Schmidt et Michel (Angew. Chem., Int. Ed. Engl., 19, 1980, 731-732) sont agitées dans 2 ml de toluène anhydre pendant 3 heures sous atmosphère inerte en présence de tamis moléculaire (4Å). Le mélange est refroidi à -78°C, et 92 µl d'éthèrate de trifluorure de bore dans le toluène sont alors ajoutés. Après retour à 0°C, le mélange est neutralisé par de l'éthyl diisopropylamine puis filtré et évaporé.

Le produit attendu, isomère 1, est purifié et séparé de l'isomère 2 par chromatographie sur gel de silice en utilisant comme éluant un mélange hexanes/acétate d'éthyle (3/1).

Pouvoir rotatoire : $[\alpha]_D^{20}$ = - 50° (C = 1 mg/ml / $CHCL_3$)

Stade B : 7-(β-D-Glucopyranosyl)benzo[c]cyclodéca-1,5-diyne, isomère 1

Le produit obtenu au stade précédent en solution dans un mélange dichlorométhane/méthanol (1/1) est traité par une quantité catalytique de méthanolate de sodium à température ambiante, sous atmosphère inerte. Le mélange réactionnel est alors neutralisé sur de la résine Amberlite (IRC 50 -forme $H^+$) puis filtré. Le produit attendu est alors obtenu après filtration, évaporation du solvant et purification par chromatographie sur gel de silice en utilisant comme éluant un mélange dichlorométhane/méthanol (6/1).

Rendement : 80 %

Point de fusion : 123°C (MeOH)

Pouvoir rotatoire : $[\alpha]_{D^{20}}$ = - 18°C (C = 0,85 mg/ml - acétone)

**EXEMPLE 9 : 7-(β-D-Glucopyranosyl)benzo[c]cyclodéca-1,5-diyne, isomére 2**

Le produit attendu est obtenu en procédant comme au stade B de l'exemple 8, à partir de l'isomère 2 du stade A de l'exemple 8.

Rendement : 80 %

Point de fusion : 126°C

Pouvoir rotatoire : $[\alpha]_D^{20}$ = + 77° (C = 1,2 mg/ml - $CHCl_3$/MeoH (1/1))

ETUDE PHARMACOLOGIQUE DES DERIVES DE L'INVENTION

**EXEMPLE 10 : Cytotoxicité in vitro**

Les cellules L 1210 (Leucémie murine) sont cultivées dans du RPMI 1640 complémenté avec 10 % de sérum de veau foetal, 2 mM de L-glutamine, 100 unités/ml de pénicilline, 100 µg/ml de streptomycine et 10 mM d'Hepes (pH : 7,4). L'inhibition de la croissance cellulaire est mesurée selon le "Microculture Tetrazolium Assay" décrit par M. ALLEY et coll. (Cancer Res., 48, 589-601, 1988).

Les résultats de ce test sont exprimés en $IC_{50}$, concentration inhibant à 50 % la croissance cellulaire. Le produit de référence utilisé est le BCNU, agent alkylant très actif utilisé en clinique.

Lors de ce test, l'$IC_{50}$ du composé de l'exemple 2 est égale à 5,6 µM, celle du composé de l'exemple 7 est égale à 0,5 µM alors que celle du BCNU est égale à 6,4 µM.

COMPOSITION PHARMACEUTIQUE

**EXEMPLE 11 : Comprimé : formule de préparation pour 1000 comprimés dosés à 2 mg**

| | |
|---|---|
| Composé de l'exemple 2 | 2 g |
| Hydroxypropylcellulose | 2 g |
| Amidon de blé | 10 g |
| Lactose | 100 g |
| Stéarate de magnésium | 3 g |
| Talc | 3 g |

## Revendications

**Revendications pour les Etats contractants suivants : AT, BE, CH, DE, DK, FR, GB, IT, LI, LU, NL, PT, SE**

**1.** Composés de formule (I) :

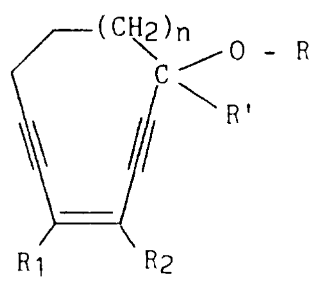

(I)

dans laquelle :

R représente un atome d'hydrogène, un radical acyl ($C_1$-$C_6$) linéaire ou ramifié ou un radical glycoside,

R' représente un atome d'hydrogène ou

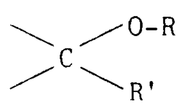

représente

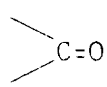

n est égal à 1 ou 2,

$R_1$ et $R_2$ représentent simultanément deux atomes d'hydrogène ou forment avec la double liaison à laquelle ils sont attachés un noyau phényle,

leurs isomères et énantiomères.

**2.** Composés de formule (I) selon la revendication 1 dans laquelle n est égal à 1.

**3.** Composé de formule (I) selon la revendication 1 qui est le 7-acétoxy-1,5-diyne-3-cyclodécène ainsi que ses énantiomères.

**4.** Procédé de préparation des composés de formule (I) selon la revendication 1 caractérisé en ce que l'on fait réagir, en milieu anhydre, sous atmosphère inerte, le (Z)-1,2-dichloroéthylène ou le 1,2-dibromobenzène avec un alcool de formule (II) :

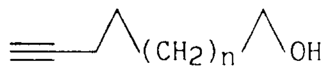

(II)

dans laquelle n a la même signification que dans la formule (I),
en présence d'un catalyseur palladié préparé à partir de tétrakis (triphénylphosphine) palladium, de n-propylamine et d'iodure de cuivre, pour conduire au composé de formule (III) :

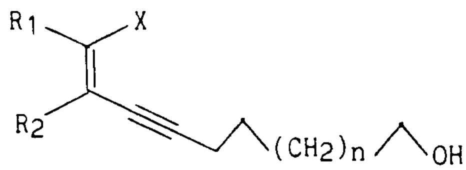

(III)

dans laquelle n, $R_1$ et $R_2$ ont la même signification que dans la formule (I) et X représente un atome de chlore ou de brome selon le produit de départ utilise,
qui est couplé, comme précédemment, en présence de tétrakis (triphénylphosphine) palladium, de n-propylamine et d'iodure de cuivre, avec du triméthylsilylacétylène, en milieu anhydre, sous atmosphère inerte,
pour conduire au composé de formule (IV) :

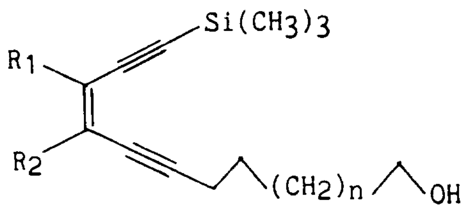

(IV)

dans laquelle n, $R_1$ et $R_2$ ont la même signification que dans la formule (I),
que l'on soumet à une désilylation en milieu basique anhydre, sous atmosphère inerte,
pour conduire au composé de formule (V) :

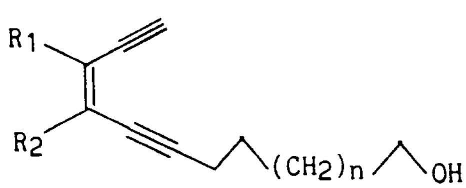

(V)

dans laquelle n, $R_1$ et $R_2$ ont la même signification que dans la formule (I),
sur lequel on fait réagir de l'iode en milieu organique anhydre en présence de morpholine, sous atmosphère inerte,
pour conduire au composé de formule (VI) :

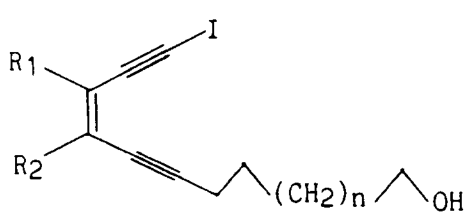

(VI)

dans laquelle n, $R_1$ et $R_2$ ont la même signification que dans la formule (I),

que l'on soumet à une oxydation en présence de chlorochromate de pyridinium en milieu organique anhydre, sous atmosphère inerte,
pour conduire au composé de formule (VII) :

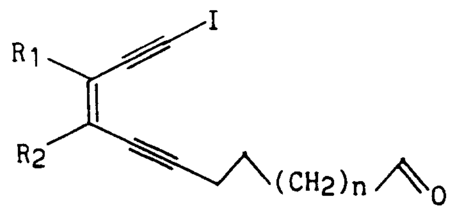

(VII)

dans laquelle n, $R_1$ et $R_2$ ont la même signification que dans la formule (I),
que l'on cyclise à l'aide d'un mélange de chlorure de chrome contenant 1,3 % de chlorure de nickel en suspension dans du tétrahydrofurane anhydre, à température ambiante, sous atmosphère inerte,
pour conduire au composé de formule (I/a), cas particulier des composés de formule (I) :

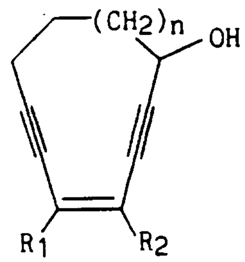

(I/a)

dans laquelle n a la même signification que dans la formule (I), qui est :

- soit transformé en ester correspondant, sous atmosphère inerte,

pour conduire au composé de formule (I/b), cas particulier des composés de formule (I),

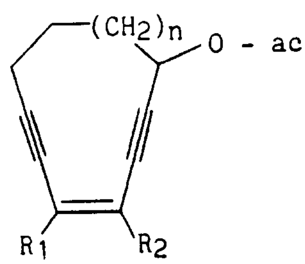

(I/b)

dans laquelle n, $R_1$ et $R_2$ ont la même signification que dans la formule (I) et ac représente un groupement acyl ($C_1$-$C_6$) linéaire ou ramifié,

- soit oxydé en présence de chlorochromate de pyridinium,

pour conduire au composé de formule (I/c), cas particulier des composés de formule (I),

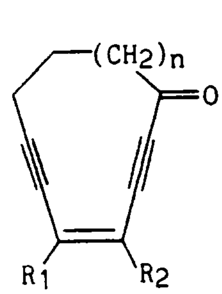

(I/c)

dans laquelle n, $R_1$ et $R_2$ ont la même signification que dans la formule (I),

- soit glycosylé en présence de trichloroacétamidate d'acétylglycoside puis désacétylé après séparation éventuelle des isomères,

pour conduire au composé de formule (I/d), cas particulier des composés de formule (I),

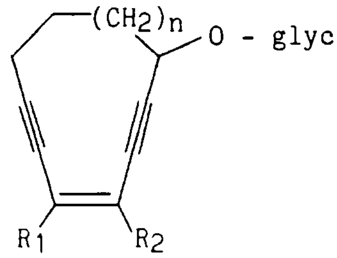

(I/d)

dans laquelle n, $R_1$ et $R_2$ ont la même signification que dans la formule (I) et glyc représente un groupement glycoside,
composés de formule (I/a), (I/b), (I/c) et (I/d) que l'on purifie, le cas échéant, selon une technique classique de préparation et dont on sépare les isomères, si on le souhaite, selon une technique classique de séparation.

**5.** Compositions pharmaceutiques contenant comme principe actif au moins un composé selon l'une quelconque des revendications 1 à 3, seul ou en combinaison avec un ou plusieurs excipients ou véhicules inertes, non toxiques, pharmaceutiquement acceptables.

**6.** Compositions pharmaceutiques selon la revendication 5 contenant au moins un principe actif selon l'une quelconque des revendications 1 à 3 utiles dans le traitement du cancer.

**Revendications pour les Etats contractants suivants : ES, GR**

**1.** Procédé de préparation des composés de formule (I) :

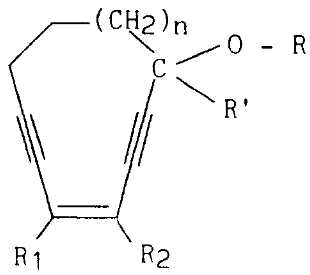

(I)

dans laquelle :

R représente un atome d'hydrogène, un radical acyl ($C_1$-$C_6$) linéaire ou ramifié ou un radical glycoside,

R' représente un atome d'hydrogène ou

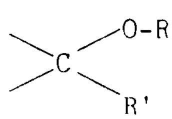

représente

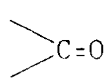

n est égal à 1 ou 2,

$R_1$ et $R_2$ représentent simultanément deux atomes d'hydrogène ou forment avec la double liaison à laquelle ils sont attachés un noyau phényle,

leurs isomères et énantiomères,
caractérise en ce que l'on fait réagir, en milieu anhydre, sous atmosphère inerte, le (Z)-1,2-dichloroéthylène ou le 1,2-dibromobenzène avec un alcool de formule (II) :

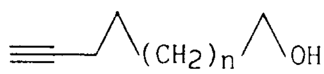

(II)

dans laquelle n a la même signification que dans la formule (I),
en présence d'un catalyseur palladié préparé à partir de tétrakis (triphénylphosphine) palladium, de n-propylamine et d'iodure de cuivre, pour conduire au composé de formule (III) :

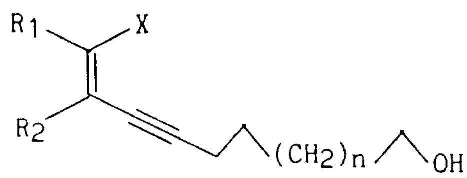

(III)

dans laquelle n, $R_1$ et $R_2$ ont la même signification que dans la formule (I) et X représente un atome de chlore ou de brome selon le produit de départ utilisé,
qui est couplé, comme précédemment, en présence de tétrakis (triphénylphosphine) palladium, de n-propylamine et d'iodure de cuivre, avec du triméthylsilylacétylène, en milieu anhydre, sous atmosphère inerte,
pour conduire au composé de formule (IV) :

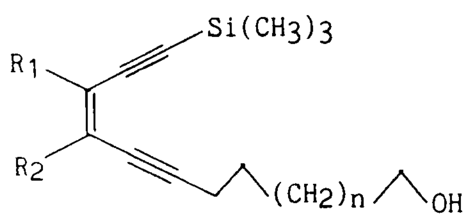

(IV)

dans laquelle n, $R_1$ et $R_2$ ont la même signification que dans la formule (I),
que l'on soumet à une désilylation en milieu basique anhydre, sous atmosphère inerte,
pour conduire au composé de formule (V) :

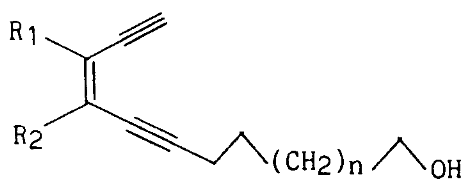

(V)

dans laquelle n, $R_1$ et $R_2$ ont la même signification que dans la formule (I),
sur lequel on fait réagir de l'iode en milieu organique anhydre en présence de morpholine, sous atmosphère inerte,
pour conduire au composé de formule (VI) :

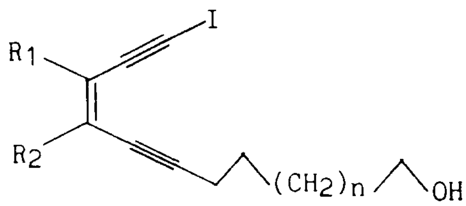

(VI)

dans laquelle n, $R_1$ et $R_2$ ont la même signification que dans la formule (I), que l'on soumet à une oxydation en présence de chlorochromate de pyridinium en milieu organique anhydre, sous atmosphère inerte,
pour conduire au composé de formule (VII) :

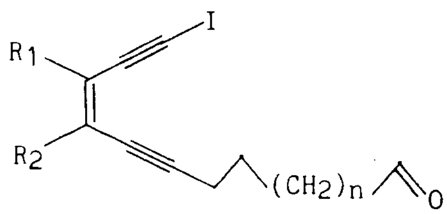

(VII)

dans laquelle n, $R_1$ et $R_2$ ont la même signification que dans la formule (I), que l'on cyclise à l'aide d'un mélange de chlorure de chrome contenant 1,3 % de chlorure de nickel en suspension dans du tétrahydrofurane anhydre, à température ambiante, sous atmosphère inerte,
pour conduire au composé de formule (I/a), cas particulier des composés de formule (I) :

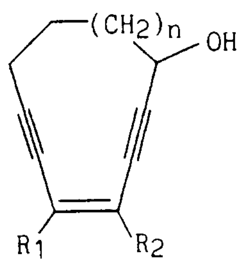

(I/a)

dans laquelle n a la même signification que dans la formule (I), qui est :

- soit transformé en ester correspondant, sous atmosphère inerte,

pour conduire au composé de formule (I/b), cas particulier des composés de formule (I),

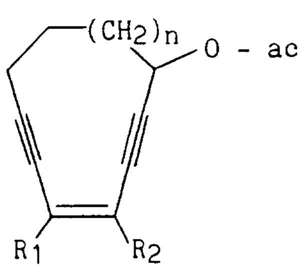

(I/b)

dans laquelle n, $R_1$ et $R_2$ ont la même signification que dans la formule (I) et ac représente un groupement acyl ($C_1$-$C_6$) linéaire ou ramifié,

- soit oxydé en présence de chlorochromate de pyridinium,

pour conduire au composé de formule (I/c), cas particulier des composés de formule (I),

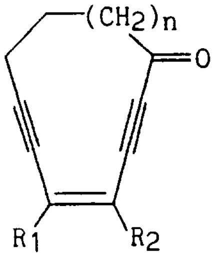

(I/c)

dans laquelle n, $R_1$ et $R_2$ ont la même signification que dans la formule (I),

- soit glycosylé en présence de trichloroacétamidate d'acétylglycoside puis désacétylé après séparation éventuelle des isomères,

pour conduire au composé de formule (I/d), cas particulier des composés de formule (I),

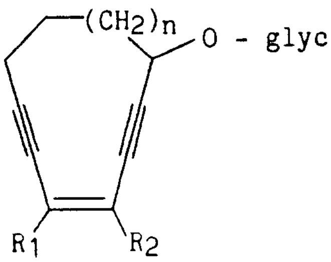

(I/d)

dans laquelle n, $R_1$ et $R_2$ ont la même signification que dans la formule (I) et glyc représente un groupement glycoside,
composés de formule (I/a), (I/b), (I/c) et (I/d) que l'on purifie, le cas échéant, selon une technique classique de préparation et dont on sépare les isomères, si on le souhaite, selon une technique classique de séparation.

**2.** Procédé de préparation des composés de formule (I) selon la revendication 1 dans laquelle n est égal à 1.

**3.** Procédé de préparation du composé de formule (I) selon la revendication 1 qui est le 7-acétoxy-1,5-diyne-3-cyclodécène ainsi que ses énantiomères.

**Claims**
**Claims for the following Contracting States : AT, BE, CH, DE, DK, FR, GB, IT, LI, LU, NL, PT, SE**

**1.** Compounds of formula (I):

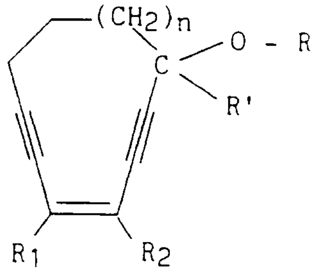

(I)

wherein:

| | |
|---|---|
| R | represents a hydrogen atom, a linear or branched ($C_1$-$C_6$)-acyl radical or a glycoside radical, |
| R' | represents a hydrogen atom or |

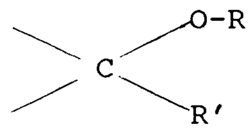

represents

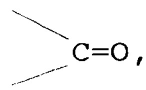

n — is 1 or 2, and

$R_1$ and $R_2$ — represent simultaneously two hydrogen atoms or form with the double bond to which they are attached a phenyl ring,

and their isomers and enantiomers.

**2.** Compounds of formula (I) according to claim 1, wherein n is 1.

**3.** Compound of formula (I) according to claim 1 that is 7-acetoxy-1,5-diyne-3-cyclodecene and also its enantiomers.

**4.** Process for the preparation of the compounds of formula (I) according to claim 1, characterised in that (Z)-1,2-dichloroethylene or 1,2-dibromobenzene is reacted in an anhydrous medium and under an inert atmosphere with an alcohol of formula (II):

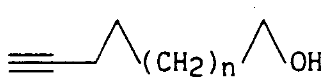

(II),

wherein n is as defined in formula (I),
in the presence of a palladium catalyst prepared from tetrakis(triphenylphosphine)palladium, n-propylamine and copper iodide, to yield the compound of formula (III):

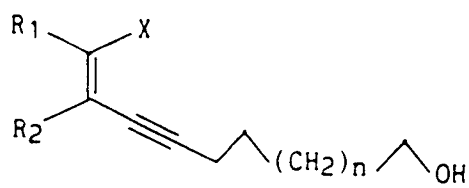

(III),

wherein n, $R_1$ and $R_2$ are as defined in formula (I) and X represents a chlorine or bromine atom, depending on the starting material used,
which is coupled, as above, in the presence of tetrakis(triphenylphosphine)palladium, n-propylamine and copper iodide with trimethylsilylacetylene in an anhydrous medium and under an inert atmosphere to yield the compound of formula (IV):

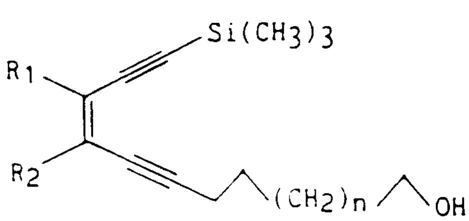

(IV),

wherein n, $R_1$ and $R_2$ are as defined in formula (I),

which is subjected to desilylation in an anhydrous basic medium and under an inert atmosphere to yield the compound of formula (V):

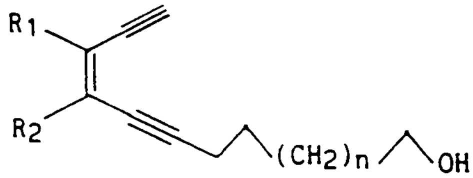

(V),

wherein n, $R_1$ and $R_2$ are as defined in formula (I), with which is reacted iodine in an anhydrous organic medium in the presence of morpholine and under an inert atmosphere to yield the compound of formula (VI):

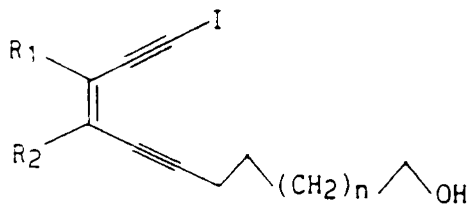

(VI),

wherein n, $R_1$ and $R_2$ are as defined in formula (I), which is subjected to oxidation in the presence of pyridinium chlorochromate in an anhydrous organic medium and under an inert atmosphere to yield the compound of formula (VII):

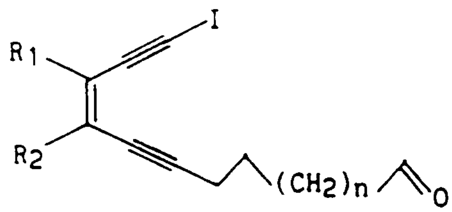

(VII),

wherein n, $R_1$ and $R_2$ are as defined in formula (I), which is cyclised with the aid of a mixture of chromium chloride containing 1.3 % of nickel chloride in suspension in anhydrous tetrahydrofuran at ambient temperature and under an inert atmosphere to yield the compound of formula (I/a), a particular case of the compounds of formula (I):

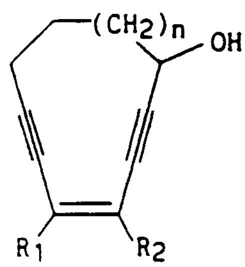

(I/a),

wherein n is as defined in formula (I), which is:

- converted into the corresponding ester under an inert atmosphere

to yield the compound of formula (I/b), a particular case of the compounds of formula (I),

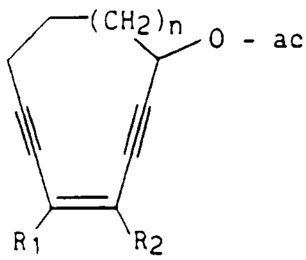

(I/b)

wherein n, $R_1$ and $R_2$ are as defined in formula (I) and ac represents a linear or branched ($C_1$-$C_6$)-acyl group,

- or oxidised in the presence of pyridinium chlorochromate

to yield the compound of formula (I/c), a particular case of the compounds of formula (I),

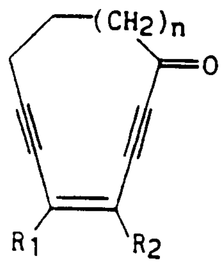

(I/c)

wherein n, $R_1$ and $R_2$ are as defined in formula (I),

- or glycosylated in the presence of acetylglycoside trichloroacetamidate and then deacetylated after optional separation of the isomers

to yield the compound of formula (I/d), a particular case of the compounds of formula (I),

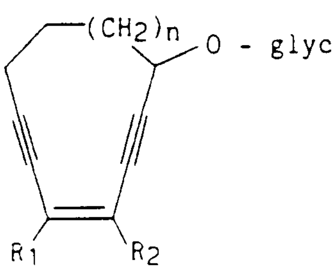

(I/d)

wherein n, $R_1$ and $R_2$ are as defined in formula (I) and glyc represents a glycoside group, which compounds of formulae (I/a), (I/b), (I/c) and (I/d) are purified, where appropriate, in accordance with a conventional preparation technique and the isomers of which are separated, if desired, in accordance with a conventional separation technique.

**5.** Pharmaceutical compositions containing as active ingredient at least one compound according to any one of claims 1 to 3, alone or in combination with one or more pharmaceutically acceptable non-toxic, inert excipients or carriers.

**6.** Pharmaceutical compositions according to claim 5 containing at least one active ingredient according to any one of claims 1 to 3 for use in the treatment of cancer.

**Claims for the following Contracting States : ES, GR**

**1.** Process for the preparation of the compounds of formula (I):

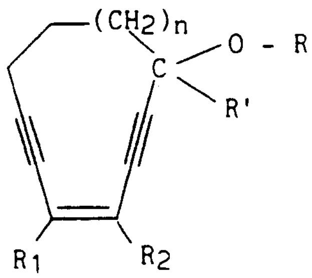

(I)

wherein:

R represents a hydrogen atom, a linear or branched ($C_1$-$C_6$)-acyl radical or a glycoside radical,

R' represents a hydrogen atom or

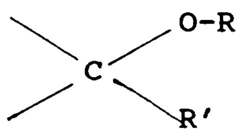

represents

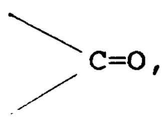

n is 1 or 2, and

$R_1$ and $R_2$ represent simultaneously two hydrogen atoms or form with the double bond to which they are attached a phenyl ring,

and their isomers and enantiomers,

characterised in that (Z)-1,2-dichloroethylene or 1,2-dibromobenzene is reacted in an anhydrous medium and under an inert atmosphere with an alcohol of formula (II):

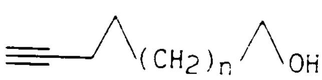

(II),

wherein n is as defined in formula (I),

in the presence of a palladium catalyst prepared from tetrakis(triphenylphosphine)palladium, n-propylamine and copper iodide, to yield the compound of formula (III):

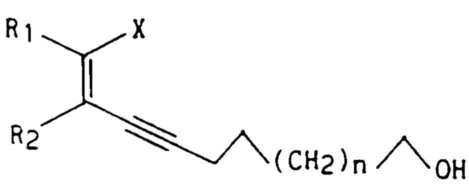

(III),

wherein n, $R_1$ and $R_2$ are as defined in formula (I) and X represents a chlorine or bromine atom, depending on the starting material used,

which is coupled, as above, in the presence of tetrakis(triphenylphosphine)palladium, n-propylamine

and copper iodide with trimethylsilylacetylene in an anhydrous medium and under an inert atmosphere to yield the compound of formula (IV):

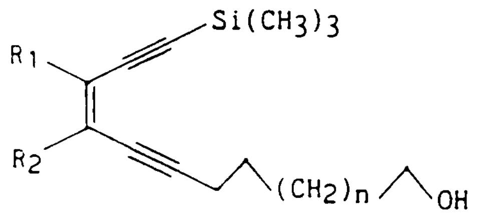

(IV),

wherein n, $R_1$ and $R_2$ are as defined in formula (I),
which is subjected to desilylation in an anhydrous basic medium and under an inert atmosphere to yield the compound of formula (V):

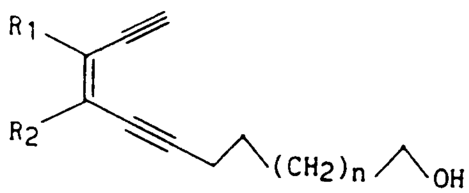

(V),

wherein n, $R_1$ and $R_2$ are as defined in formula (I),
with which is reacted iodine in an anhydrous organic medium in the presence of morpholine and under an inert atmosphere
to yield the compound of formula (VI):

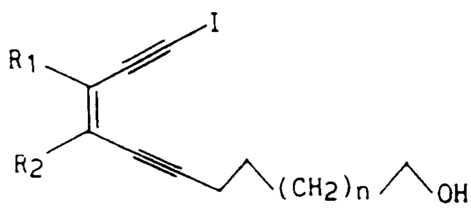

(VI),

wherein n, $R_1$ and $R_2$ are as defined in formula (I),
which is subjected to oxidation in the presence of pyridinium chlorochromate in an anhydrous organic medium and under an inert atmosphere
to yield the compound of formula (VII):

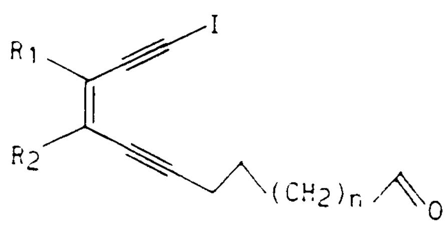

(VII),

wherein n, $R_1$ and $R_2$ are as defined in formula (I),
which is cyclised with the aid of a mixture of chromium chloride containing 1.3 % of nickel chloride in suspension in anhydrous tetrahydrofuran at ambient temperature and under an inert atmosphere
to yield the compound of formula (I/a), a particular case of the compounds of formula (I):

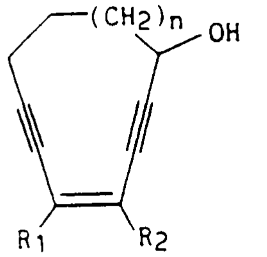

(I/a),

wherein n is as defined in formula (I), which is:

- converted into the corresponding ester under an inert atmosphere

to yield the compound of formula (I/b), a particular case of the compounds of formula (I),

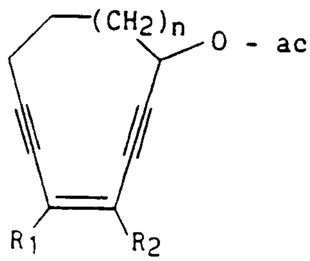

(I/b)

wherein n, $R_1$ and $R_2$ are as defined in formula (I) and ac represents a linear or branched ($C_1$-$C_6$)-acyl group,

- or oxidised in the presence of pyridinium chlorochromate

to yield the compound of formula (I/c), a particular case of the compounds of formula (I),

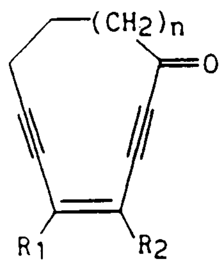

(I/c)

wherein n, $R_1$ and $R_2$ are as defined in formula (I),

- or glycosylated in the presence of acetylglycoside trichloroacetamidate and then deacetylated after optional separation of the isomers

to yield the compound of formula (I/d), a particular case of the compounds of formula (I),

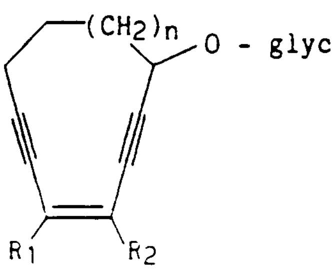

(I/d)

wherein n, $R_1$ and $R_2$ are as defined in formula (I) and glyc represents a glycoside group, which compounds of formulae (I/a), (I/b), (I/c) and (I/d) are purified, where appropriate, in accordance with a conventional preparation technique and the isomers of which are separated, if desired, in accordance with a conventional separation technique.

**2.** Process for the preparation of the compounds of formula (I) according to claim 1, wherein n is 1.

**3.** Process for the preparation of the compound of formula (I) according to claim 1 that is 7-acetoxy-1,5-diyne-3-cyclodecene and also its enantiomers.

**Patentansprüche**
**Patentansprüche für folgende Vertragsstaaten : AT, BE, CH, DE, FR, GB, IT, LI, LU, NL, PT, SE**

**1.** Verbindungen der Formel (I):

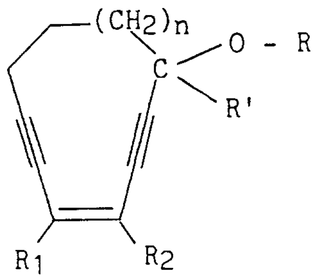

(I)

in der:

R ein Wasserstoffatom, eine geradkettige oder verzweigte ($C_1$-$C_6$)-Acylgruppe oder eine Glykosidgruppe,

R' ein Wasserstoffatom oder

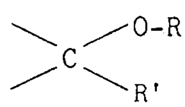

den Rest

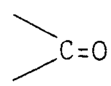

n 1 oder 2 und

$R_1$ und $R_2$ gleichzeitig zwei Wasserstoffatome oder zusammen mit der Doppelbindung, an die sie gebunden sind, einen Phenylrest bedeuten,

und deren Isomere und Enantiomere.

**2.** Verbindungen der Formel (I) nach Anspruch 1, worin n den Wert 1 besitzt.

**3.** Verbindung der Formel (I) nach Anspruch 1, nämlich 7-Acetoxy-1,5-diin-3-cyclodecen sowie dessen Enantiomere.

**4.** Verfahren zur Herstellung der Verbindungen der Formel (I) nach Anspruch 1, **dadurch gekennzeichnet**, daß man (Z)-1,2-Dichlorethylen oder 1,2-Dibrombenzol in wasserfreiem Medium und unter inerter Atmosphäre mit einem Alkohol der Formel (II):

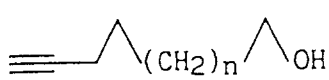

(II)

in der n die bezuglich der Formel (I) angegebenen Bedeutungen besitzt, in Gegenwart eines ausgehend

von Tetrakis(triphenylphosphin)-palladium, n-Propylamin und Kupferiodid hergestellten Palladiumkatalysators umsetzt, zur Bildung der Verbindung der Formel (III):

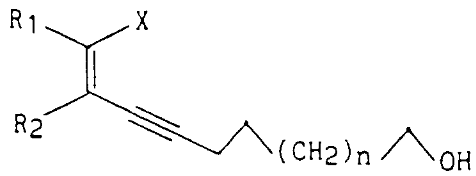

(III)

in der n, $R_1$ und $R_2$ die bezüglich der Formel (I) angegebenen Bedeutungen besitzen und X in Abhängigkeit von dem verwendeten Ausgangsmaterial ein Chlor- oder Bromatom darstellt,
welche anschließend in der oben angegebenen Weise in wasserfreiem Medium und unter inerter Atmosphäre in Gegenwart von Tetrakis(triphenylphosphin)-palladium, n-Propylamin und Kupferiodid mit Trimethylsilylacetylen gekuppelt wird,
zur Bildung der Verbindung der Formel (IV):

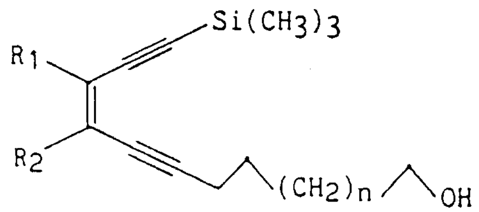

(IV)

in der n, R1 und $R_2$ die bezüglich der Formel (I) angegebenen Bedeutungen besitzen,
welche man unter einer inerten Atmosphäre in wasserfreiem basischem Medium einer Desilylierung unterwirft,
so daß man die Verbindung der Formel (V):

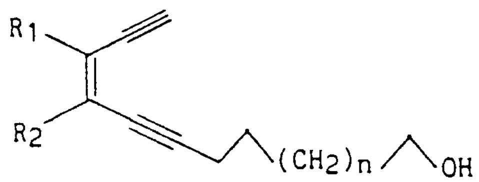

(V)

erhält, in der n, $R_1$ und $R_2$ die bezüglich der Formel (I) angegebenen Bedeutungen besitzen,
welche man in wasserfreiem organischem Medium und in Gegenwart von Morpholin unter inerter Atmosphäre mit Iod umsetzt,
zur Bildung der Verbindung der Formel (VI):

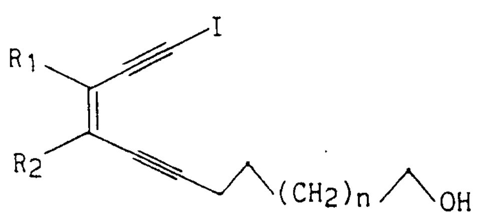

(VI)

in der n, $R_1$ und $R_2$ die bezüglich der Formel (I) angegebenen Bedeutungen besitzen,
welche man in Gegenwart von Pyridinium-chlorochromat in wasserfreiem organischem Medium unter inerter Atmosphäre einer Oxidation unterwirft,
zur Bildung der Verbindung der Formel (VII):

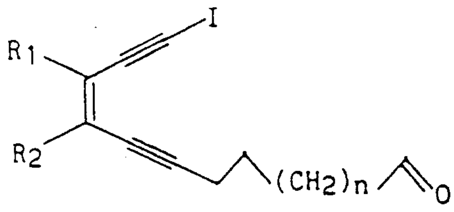

(VII)

in der n, $R_1$ und $R_2$ die bezüglich der Formel (I) angegebenen Bedeutungen besitzen, welche man in Suspension in wasserfreiem Tetrahydrofuran bei Raumtemperatur unter inerter Atmosphäre mit Hilfe einer Mischung von Chromchlorid, die 1,3 % Nickelchlorid enthält, cyclisiert, zur Bildung der Verbindung der Formel (I/a), einem Sonderfall der Verbindungen der Formel (I):

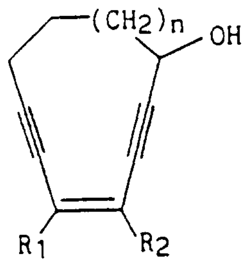

(I/a)

in der n die bezüglich der Formel (I) angegebenen Bedeutungen besitzt, welche

- entweder unter inerter Atmosphäre in den entsprechenden Ester umgewandelt wird,

zur Bildung der Verbindung der Formel (I/b), einem Sonderfall der Verbindungen der Formel (I):

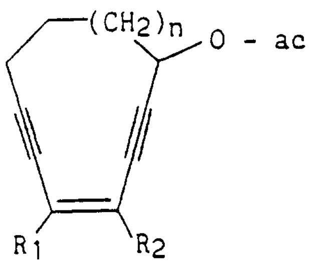

(I/b)

in der n, $R_1$ und $R_2$ die bezüglich der Formel (I) angegebenen Bedeutungen besitzen und ac eine geradkettige oder verzweigte $(C_1\text{-}C_6)$-Acylgruppe darstellt,

- oder in Gegenwart von Pyridinium-chlorochromat oxidiert wird,

zur Bildung der Verbindung der Formel (I/c), einem Sonderfall der Verbindungen der Formel (I):

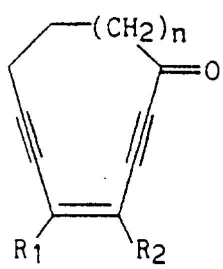

(I/c)

in der n, $R_1$ und $R_2$ die bezüglich der Formel (I) angegebenen Bedeutungen besitzen,

- oder in Gegenwart von Acetylglykosid-trichloroacetamidat glykosyliert und nach der eventuellen Trennung der Isomeren desacetyliert wird,

zur Bildung der Verbindung der Formel (I/d), einem Sonderfall der Verbindungen der Formel (I):

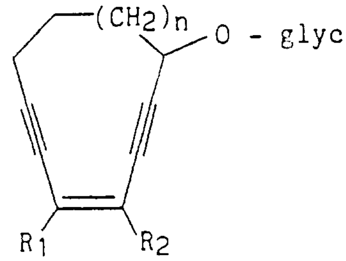

(I/d)

in der n, $R_1$ und $R_2$ die bezüglich der Formel (I) angegebenen Bedeutungen besitzen und glyc eine Glykosidgruppe bedeutet,
welche Verbindungen der Formel (I/a), (I/b) (I/c) und (I/d) man gegebenenfalls mit Hilfe einer klassischen Reinigungstechnik reinigt und deren Isomeren man gewünschtenfalls mit Hilfe einer klassischen Trennmethode trennt.

**5.** Pharmazeutische Zubereitungen enthaltend als Wirkstoff mindestens eine Verbindung nach einem der Ansprüche 1 bis 3 allein oder in Kombination mit einem oder mehreren inerten, nichttoxischen, pharmazeutisch annehmbaren Trägermaterialien oder Bindemitteln.

**6.** Pharmazeutische Zubereitungen nach Anspruch 5, enthaltend mindestens einen Wirkstoff nach einem der Ansprüche 1 bis 3, für die Behandlung von Krebs.

**Patentansprüche für folgende Vertragsstaaten : ES, GR**

**1.** Verfahren zur Herstellung der Verbindungen der Formel (I):

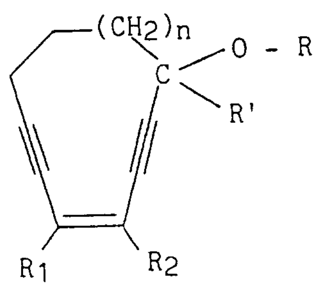

(I)

in der:

| | |
|---|---|
| R | ein Wasserstoffatom, eine geradkettige oder verzweigte ($C_1$-$C_6$)-Acylgruppe oder eine Glykosidgruppe, |
| R' | ein Wasserstoffatom oder |

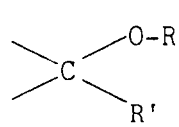

den Rest

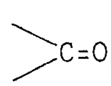

| | |
|---|---|
| n | 1 oder 2 und |
| $R_1$ und $R_2$ | gleichzeitig zwei Wasserstoffatome oder zusammen mit der Doppelbindung, an die sie gebunden sind, einen Phenylrest bedeuten, |

und von deren Isomeren und Enantiomeren,
**dadurch gekennzeichnet**, daß man (Z)-1,2-Dichlorethylen oder 1,2-Dibrombenzol in wasserfreiem Medium und unter inerter Atmosphäre mit einem Alkohol der Formel (II):

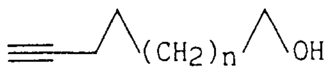

(II)

in der n die bezüglich der Formel (I) angegebenen Bedeutungen besitzt,
in Gegenwart eines ausgehend von Tetrakis(triphenylphosphin)-palladium, n-Propylamin und Kupferiodid hergestellten Palladiumkatalysators umsetzt,
zur Bildung der Verbindung der Formel (III):

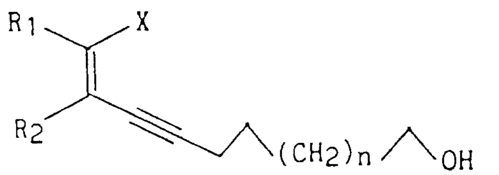

(III)

in der n, $R_1$ und $R_2$ die bezüglich der Formel (I) angegebenen Bedeutungen besitzen und X in Abhängigkeit von dem verwendeten Ausgangsmaterial ein Chlor- oder Bromatom darstellt,
welche anschließend in der oben angegebenen Weise in wasserfreiem Medium und unter inerter Atmosphäre in Gegenwart von Tetrakis(triphenylphosphin)-palladium, n-Propylamin und Kupferiodid mit Trimethylsilylacetylen gekuppelt wird,
zur Bildung der Verbindung der Formel (IV):

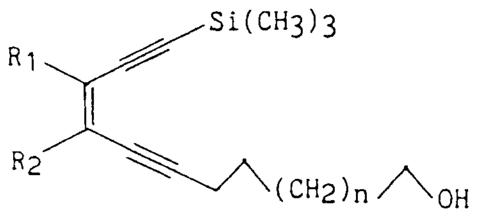

(IV)

in der n, $R_1$ und $R_2$ die bezüglich der Formel (I) angegebenen Bedeutungen besitzen,
welche man unter einer inerten Atmosphäre in wasserfreiem basischem Medium einer Desilylierung unterwirft,
so daß man die Verbindung der Formel (V):

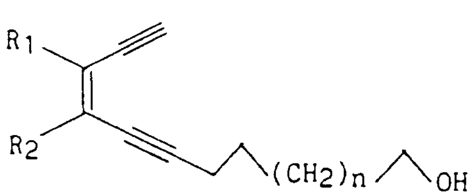

(V)

erhält, in der n, $R_1$ und $R_2$ die bezüglich der Formel (I) angegebenen Bedeutungen besitzen,
welche man in wasserfreiem organischem Medium und in Gegenwartvon Morpholin unter inerter Atmosphäre mit Iod umsetzt,
zur Bildung der Verbindung der Formel (VI):

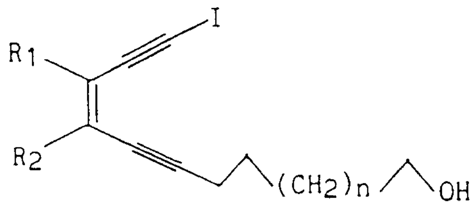

(VI)

in der n, $R_1$ und $R_2$ die bezüglich der Formel (I) angegebenen Bedeutungen besitzen,
welche man in Gegenwartvon Pyridinium-chlorochromat in wasserfreiem organischem Medium unter inerter Atmosphäre einer Oxidation unterwirft,
zur Bildung der Verbindung der Formel (VII):

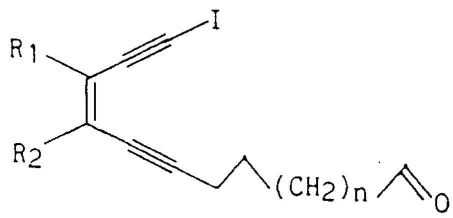

(VII)

in der n, $R_1$ und $R_2$ die bezüglich der Formel (I) angegebenen Bedeutungen besitzen,
welche man in Suspension in wasserfreiem Tetrahydrofuran bei Raumtemperatur unter inerter Atmosphäre mit Hilfe einer Mischung von Chromchlorid, die 1,3 % Nickelchlorid enthält, cyclisiert,
zur Bildung der Verbindung der Formel (I/a), einem Sonderfall der Verbindungen der Formel (I):

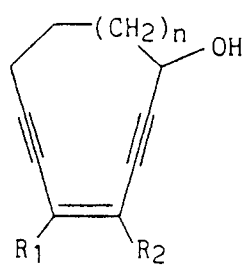

(I/a)

in der n die bezüglich der Formel (I) angegebenen Bedeutungen besitzt, welche

- entweder unter inerter Atmosphäre in den entsprechenden Ester umgewandelt wird,

zur Bildung der Verbindung der Formel (I/b), einem Sonderfall der Verbindungen der Formel (I):

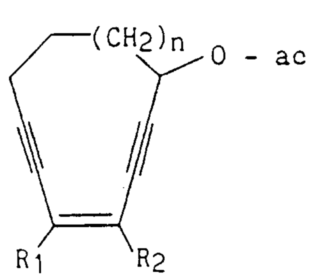

(I/b)

in der n, $R_1$ und $R_2$ die bezüglich der Formel (I) angegebenen Bedeutungen besitzen und ac eine geradkettige oder verzweigte ($C_1$-$C_6$)-Acylgruppe darstellt,

- oder in Gegenwart von Pyridinium-chlorochromat oxidiert wird,

zur Bildung der Verbindung der Formel (I/c), einem Sonderfall der Verbindungen der Formel (I):

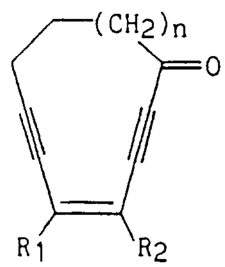

(I/c)

in der n, $R_1$ und $R_2$ die bezüglich der Formel (I) angegebenen Bedeutungen besitzen,

- oder in Gegenwart von Acetylglykosid-trichloroacetamidat glykosyliert und nach der eventuellen Trennung der Isomeren desacetyliert wird,

zur Bildung der Verbindung der Formel (I/d), einem Sonderfall der Verbindungen der Formel (I):

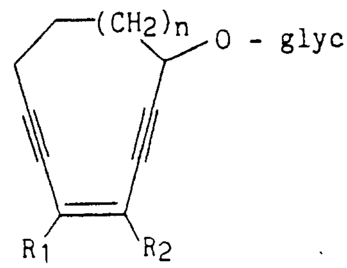

(I/d)

in der n, $R_1$ und $R_2$ die bezüglich der Formel (I) angegebenen Bedeutungen besitzen und glyc eine Glykosidgruppe bedeutet,
welche Verbindungen der Formel (I/a), (I/b), (I/c) und (I/d) man gegebenenfalls mit Hilfe einer klassischen Reinigungstechnik reinigt und deren Isomeren man gewünschtenfalls mit Hilfe einer klassischen Trennmethode trennt.

**2.** Verfahren zur Herstellung der Verbindungen der Formel (I) nach Anspruch 1, worin n den Wert 1 besitzt.

**3.** Verfahren zur Herstellung der Verbindung der Formel (I) nach Anspruch 1, nämlich von 7-Acetoxy-1,5-diin-3-cyclodecen und dessen Enantiomeren.